# EUROPEAN PATENT APPLICATION

(11) **EP 0 650 957 A1**
(43) Date of publication of application: **03.05.1995**
(21) Application number: 93922051.3
(22) Date of filing: 07.10.1993
(51) Int. Cl.: C07C 219/34, C07C 213/02

(54) **ANILINE DERIVATIVE AND PROCESS FOR PRODUCING THE SAME**

(30) Priority: 22.03.1993 JP 61759/93
(71) Applicant: SUMITOMO CHEMICAL COMPANY LIMITED, Osaka-shi, Osaka 541 (JP)
(72) Inventor: GOTO, Hideyuki, Takatsuki-shi, Osaka 569 (JP); OHTSUKA, Susumu, Nishinomiya-shi, Hyogo 663 (JP); TAKEMOTO, Ichiki, Kawanishi-shi, Hyogo 666 (JP)
(74) Representative: VOSSIUS & PARTNER
(86) International application number: JP9301446
(87) International publication number: WO9421597

(57) **Abstract**

An aniline derivative represented by chemical formula (I); and a process for producing 5-amino-2-chloro-4-fluorophenyl ethyl carbonate by reducing 2-chloro-4-fluoro-5-nitrophenyl ethyl carbonate.

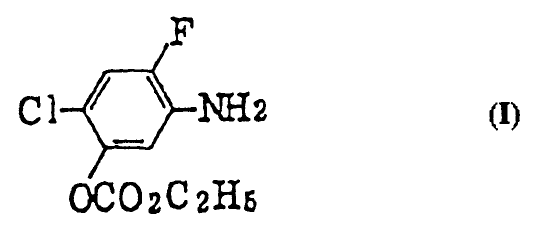

## Description

### Technical Field

The present invention relates to an aniline derivative useful as an intermediate of agricultural chemicals or the like and a process of its production.

### Background Art

It has hitherto been known that 5-(3-butyn-2-yloxy)-4-chloro-2-fluoroaniline is useful as an intermediate of agricultural chemicals, and it has also been known that this compound can be produced from (5-amino-2-chloro-4-fluorophenyl)methyl carbonate. The (5-amino-2-chloro-4-fluorophenyl)methyl carbonate can be obtained by the nitration and subsequent reduction of (2-chloro-4-fluorophenyl)methyl carbonate which is obtained by the reaction of 2-chloro-4-fluorophenol with halocarbonic acid methyl ester (JP-A 310855/1988, EP-0,061,741 and JP-A 42042/1990).

In this process, however, there is a problem in the operating characteristics because (2-chloro-4-fluorophenyl)methyl carbonate as the starting material is in the form of crystals (m.p.: 69-71°C) at ordinary temperature and it requires complicated handling operations in the reaction step; therefore, this process cannot be said to be a favorable process from an industrial point of view.

Accordingly, there has been a demand for the development of a process for producing 5-(3-butyn-2-yloxy)-4-chloro-2-fluoroaniline by no way of (2-chloro-4-fluorophenyl)methyl carbonate.

The present inventors have studied such a production process and found (2-chloro-4-fluorophenyl)ethyl carbonate having an extremely low melting point and in oil form at ordinary temperature, which is therefore easy to handle. Further, they have also found that this compound can easily be converted into (5-amino-2-chloro-4-fluorophenyl)ethyl carbonate by its nitration and subsequent reduction with good selectivity and (5-amino-2-chloro-4-fluorophenyl)ethyl carbonate can easily be led to 5-(3-butyn-2-yloxy)-4-chloro-2-fluoroaniline with good selectivity in the same manner as the conventional compound, thereby completing the present invention.

### Disclosure of the Invention

That is, the present invention provides an aniline derivative of the chemical formula [I]: 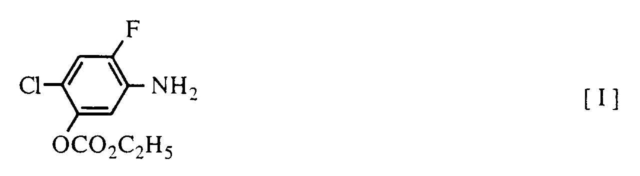
and a process of its production.

The following will fully describe the present invention.

The aniline derivative of the chemical formula [I] as the desired compound of the present invention can be obtained by the reduction of a nitrobenzene derivative of the chemical formula [II]: 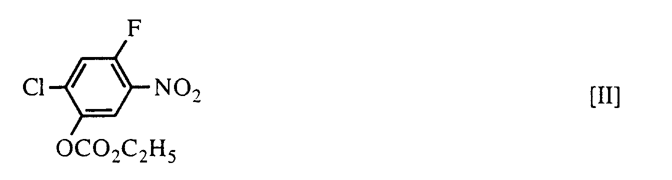
As the method for the reduction of the nitrobenzene derivative, there can be mentioned catalytic hydrogenation or chemical reduction, and the reduction may be carried out by either of these methods.

In the catalytic hydrogenation, platinum-carbon, platinum dioxide, palladium-carbon or the like is used as the catalyst, and the amount for its use is from a catalytic amount to 40% by weight, based on the nitrobenzene derivative of the chemical formula [II] as the starting material.

Also in the catalytic hydrogenation, there are some cases where corrosive hydrogen fluoride may be formed as a by-product in the reaction system, and the catalytic hydrogenation can also be carried out in the co-existence of an alkali to protect the reaction vessel. As the alkali, there can be mentioned sodium salts and potassium salts, such as sodium carbonate and potassium carbonate, and basic calcium salts such as calcium hydroxide and calcium carbonate. Among these, basic calcium salts such as calcium hydroxide and calcium carbonate are particularly preferred. The amount of the basic calcium salts to be added is usually 0.1-10 mol%, based on the nitrobenzene derivative.

The catalytic hydrogenation is usually carried out in a solvent. As the solvent, there can be exemplified, for example, organic solvents, e.g., lower alcohols such as methanol, ethanol and isopropanol, esters such as ethyl acetate, ethers such as tetrahydrofuran, and aromatic hydrocarbons such as toluene and xylene, or water, or mixtures thereof.

The hydrogen pressure of hydrogen used in the catalytic hydrogenation may be either ordinary pressure or increased pressure. The reaction temperature is usually from room temperature to 150°C.

In the chemical reduction, the reduction is usually carried out in the presence of iron powder and an acid.

In this method, the amount of iron powder to be used is 2 times or more, preferably 2 to 5 times, the mole of the nitrobenzene derivative of the chemical formula [II] as the starting material. As the acid, inorganic acids such as hydrochloric acid and sulfuric acid or organic acids such as acetic acid are used.

The chemical reduction is usually carried out in a solvent. As the solvent, there can be exemplified water, acetic acid, methanol, ethanol, isopropanol, tetrahydrofuran and mixtures thereof.

The reaction is generally carried out under ordinary pressure, and the reaction temperature is usually from room temperature to 200°C, preferably 40 to 200°C.

After completion of the reaction, the desired aniline derivative can be obtained by filtering off the catalyst and distilling off the solvent in the case of catalytic hydrogenation, or by filtering off the reducing agent used, adjusting the pH of the reaction mixture, extracting the desired product with a water-immiscible solvent, and distilling off the organic solvent in the case of chemical reduction.

The following will fully describe a process for the production of the nitrobenzene derivative of the chemical formula [II] as the staring material of the above reaction.

The nitrobenzene derivative of the chemical formula [II] can be obtained by the nitration of (2-chloro-4-fluorophenyl)ethyl carbonate which is obtained by the reaction of 2-chloro-4-fluorophenol and ethyl chlorocarbonate in an aqueous solvent.

In the reaction of 2-chloro-4-fluorophenol and ethyl chlorocarbonate, the ethyl chlorocarbonate is usually used in an equivalent which is 1 to 3 times, preferably 1 to 2 times, the equivalent of the 2-chloro-4-fluorophenol.

In this reaction, a base is usually used. As the base, there can be mentioned, for example, alkali metal hydroxides such as sodium hydroxide and potassium hydroxide, alkali metal carbonates such as potassium carbonate and sodium carbonate, or organic amines such as triethylamine, pyridine and N,N-diethylaniline. These bases can also be used as a mixture. The amount of these bases to be used is usually 1 to 3 times, preferably 1 to 2 times, the equivalent of the 2-chloro-4-fluorophenol.

Also in the above reaction, there are some cases where acceleration can be attained by the coexistence of a catalyst. As the catalyst, there can be mentioned, for example, quaternary ammonium salts such as tetrabutylammonium bromide and benzyltriethylammonium chloride, crown ethers such as 18-crown-6, phosphonium salts such as cetyltributylphosphonium bromide, or phase transfer catalysts such as tris(3,6-dioxaheptyl)amine (TDA-1). The amount of the catalyst to be used is 0.01 to 0.2 times the equivalent of the 2-chloro-4-fluorophenol.

This reaction is usually carried out in an aqueous solvent, but it can also be carried out in an organic solvent. As the organic solvent, there can be mentioned, for example, organic solvents such as toluene, xylene, methylene chloride, chloroform, 1,2-dichloroethane and monochlorobenzene. The reaction temperature is usually 0 to 100°C, preferably 0 to 50°C. Also it is desirable that the reaction is carried out while the pH of the reaction mixture is usually adjusted to pH 5 to 12, preferably pH 7 to 10.

As the reagent for the nitration of the (2-chloro-4-fluorophenyl)ethyl carbonate thus obtained, there can be mentioned, for example, nitric acid. The nitric acid used is usually in an equivalent which is 1 to 8 times, preferably 1 to 2 times, the equivalent of the starting material.

This reaction is usually carried out in a solvent. As the solvent, there can be mentioned, for example, halogenated hydrocarbons such as methylene chloride, chloroform and 1,2-dichloroethane, other organic solvents such as nitrobenzene and acetic acid, sulfuric acid, or mixtures thereof.

The reaction temperature is usually -50 to 100°C, preferably -20 to 50°C.

After completion of the reaction, the reaction mixture is diluted with ice water or the like while cooling, and if necessary, it is extracted with an organic solvent such as toluene, which makes it possible to obtain the nitrobenzene derivative in solution form. Further, the nitrobenzene derivative can be isolated by distilling off the organic solvent under reduced pressure.

According to the present invention, the aniline derivative of the chemical formula [I] as a novel compound can easily be obtained in high yield from the starting material nitrobenzene derivative [II]. Also this aniline derivative has extremely high utility value such as the use of an intermediate for the production of agricultural chemicals, and for example, it can be utilized for the production of agricultural chemicals through the following route. 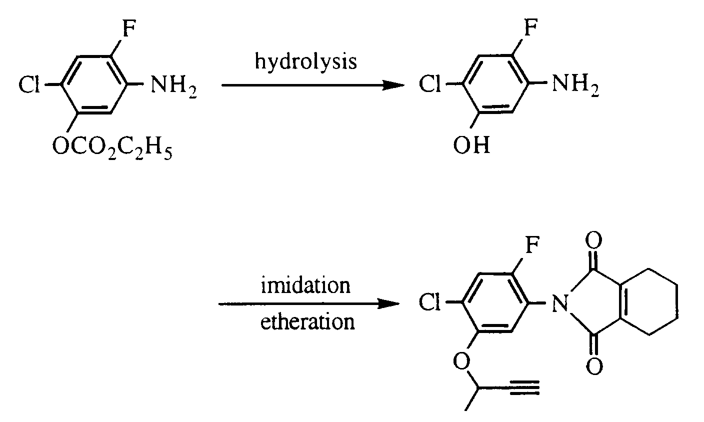
(agricultural chemical described in JP-A 38256/1983)
The present invention will be further illustrated by the following examples; however, the present invention is not limited to the following examples.

### Example 1

### Synthesis of (5-amino-2-chloro-4-fluorophenyl)ethyl carbonate

In a 500-ml autoclave charged with 20.0 g of (2-chloro-4-fluoro-5-nitrophenyl)ethyl carbonate, 60 g of toluene and 0.2 g of 5% Pd-C, the atmosphere of which was substituted with nitrogen, the reaction was carried out for 2.75 hours under the conditions that the hydrogen pressure was 20 kg/cm² and the reaction temperature was 40°C. After completion of the reaction, the reaction mixture was cooled, filtered and then concentrated, which afforded 17.48 g of (5-amino-2-chloro-4-fluorophenyl)ethyl carbonate. (Yield 95.8%)
m.p., 46-48°C
FD-MS: 233 (M⁺)
NMR (CDCl₃) δ: 1.39 (t, 3H, J = 7.0 Hz), 3.4-3.7 (broad, 2H), 4.33 (q, 2H, J = 7.0 Hz), 6.62 (d, 1H, J = 8.2 Hz), 7.07 (d, 1H, J = 10.6 Hz)

### Example 2

### Synthesis of (5-amino-2-chloro-4-fluorophenyl)ethyl carbonate

In a 500-ml autoclave were charged 26.36 g of (2-chloro-4-fluoro-5-nitrophenyl)ethyl carbonate, 79.07 g of toluene and 0.053 g of 1% Pt-C, the atmosphere of which was then substituted with nitrogen, and the reaction was carried out for 6 hours under the conditions that the hydrogen pressure was 20 kg/cm² and the reaction temperature was 80°C. After completion of the reaction, the reaction mixture was cooled, filtered and then concentrated, which afforded 23.87 g of (5-amino-2-chloro-4-fluorophenyl)ethyl carbonate. (Yield 94.1%)

### Example 3

### Synthesis of (5-amino-2-chloro-4-fluorophenyl)ethyl carbonate

In a 1000-ml autoclave were charged 1.77 g of 1% Pt-C and 39.5 g of toluene, the atmosphere of which was then substituted with nitrogen, and a toluene solution containing 79 g of (2-chloro-4-fluoro-5-nitrophenyl)ethyl carbonate was injected over 4 hours under the conditions that the hydrogen pressure was 20 kg/cm² and the reaction temperature was 80°C. After completion of the injection, maturing was carried out for 4 hours.

After completion of the reaction, the reaction mixture was cooled, filtered and then concentrated, which afforded 68.89 g of (5-amino-2-chloro-4-fluorophenyl)ethyl carbonate. (Yield 95.0%)

### Example 4

### Synthesis of (5-amino-2-chloro-4-fluorophenyl)ethyl carbonate

In a 1000-ml autoclave were charged 1.77 g of 1% Pt-C, 80.0 g of toluene and 66 mg of calcium hydroxide, the atmosphere of which was then substituted with nitrogen, and 276 g of a toluene solution containing 79 g of (2-chloro-4-fluoro-5-nitrophenyl)ethyl carbonate was injected over 4 hours under the conditions that the hydrogen pressure was 10 kg/cm² and the reaction temperature was 80°C. After completion of the injection, maturing was carried out for 4 hours. Fluoride ion in the water produced by the reduction was in 16 ppm.

After completion of the reaction, the reaction mixture was cooled, filtered and then concentrated, which afforded 69.59 g of (5-amino-2-chloro-4-fluorophenyl)ethyl carbonate. (Yield 99.4%)

### Comparative Example 1

### Synthesis of (5-amino-2-chloro-4-fluorophenyl)ethyl carbonate

In a 1000-ml autoclave were charged 1.77 g of 1% Pt-C, 80.0 g of toluene and 95 mg of sodium carbonate, the atmosphere of which was then substituted with nitrogen, and 276 g of a toluene solution containing 79 g of (2-chloro-4-fluoro-5-nitrophenyl)ethyl carbonate was injected over 4 hours under the conditions that the hydrogen pressure was 10 kg/cm² and the reaction temperature was 80°C. After completion of the injection, maturing was carried out for 4 hours. Fluoride ion in the water produced by the reduction was in 900 ppm.

After completion of the reaction, the reaction mixture was cooled, filtered and then concentrated, which afforded 68.82 g of (5-amino-2-chloro-4-fluorophenyl)ethyl carbonate. (Yield 98.3%)

### Reference Example 1

### Synthesis of (2-chloro-4-fluorophenyl)ethyl carbonate

In a four-necked flask were charged 132 g of 2-chloro-4-fluorophenol and 370 g of water, and the pH was adjusted to 7.0 by the addition of 37.0% aqueous sodium hydroxide solution in a small amount. After this was cooled in a water bath, 102.6 g of ethyl chlorocarbonate and 102.2 g of 37% sodium hydroxide were poured in parallel at a reaction temperature below 30°C over 1 hour. This reaction mixture was stirred at room temperature for 2 hours, followed by separating operation, which afforded 195 g of (2-chloro-4-fluorophenyl)ethyl carbonate as the oil layer. (Yield 99%).
m.p., 28-30°C
b.p., 98-100°C (1.7 mmHg)
δ (CDCl₃) 1.40 (3H, t, J = 7.3 Hz), 4.34 (2H, q, J = 7.3 Hz), 7.00 (1H, m), 7.22 (2H, m)
FD-MS M⁺ 218

### Reference Example 2

### Synthesis of (2-chloro-4-fluoro-5-nitrophenyl)ethyl carbonate

In a four-necked flask were charged 80 g of (2-chloro-4-fluorophenyl)ethyl carbonate and 160 g of 97% sulfuric acid, and after cooling in an ice bath, 24.5 g of 99% nitric acid was added dropwise at a reaction temperature below 30°C over 30 minutes. This reaction mixture was stirred at room temperature (20.0°C) for 2 hours, followed by cooling in an ice bath, and 120 ml of water was added dropwise below 30°C. To this was added 240 g of toluene to carry out extraction, followed by separation. The toluene layer obtained was successively washed with 250 ml of water and 5% aqueous sodium carbonate solution. From the organic layer obtained by the separation, toluene was distilled off under reduced pressure, which afforded 93.3 g of (2-chloro-4-fluoro-5-nitrophenyl)ethyl carbonate. (Yield 96.7%)
m.p., 51-53°C
NMR δ (CDCl₃) 1.43 (3H, t, J = 7.3 Hz), 4.40 (2H, q, J = 7.3 Hz), 7.46 (1H, d, J = 9.90 Hz), 8.05 (1H, d, J = 6.9 Hz)
FD-MS M⁺ 263

### Reference Example 3

### Synthesis of (2-chloro-4-fluoro-5-nitrophenyl)ethyl carbonate

In a four-necked flask were charged 280 g of (2-chloro-4-fluorophenyl)ethyl carbonate and 576 g, and after cooling in an ice bath, 189 g of a mixed acid (nitric acid/sulfuric acid, 45/50) was added dropwise at a reaction temperature below 15°C over 10 hours. This reaction mixture was stirred at room temperature (20.0°C) for 1 hour, followed by cooling in an ice bath, and 300 g of water was added dropwise below 15°C. To this was added 600 g of toluene to extract the product, followed by separation. Then, the toluene layer obtained by the separation was successively washed with 300 g of water and 300 g of 5% aqueous sodium carbonate solution. The washed organic layer was concentrated under reduced pressure, which afforded 294 g of (2-chloro-4-fluoro-5-nitrophenyl)ethyl carbonate. (Yield 91.8%)

## Claims

1. An aniline derivative of the chemical formula [I]:

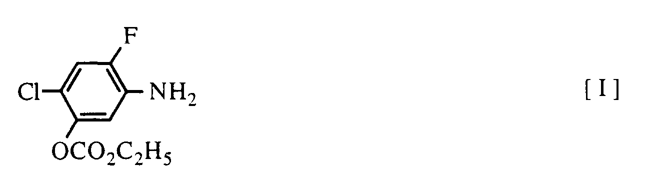

2. A process for the production of an aniline derivative of the chemical formula [I] according to claim 1, characterized in that (2-chloro-4-fluoro-5-nitrophenyl)ethyl carbonate is reduced.

3. A process for the production of an aniline derivative of the chemical formula [I] according to claim 1, characterized in that (2-chloro-4-fluoro-5-nitrophenyl)ethyl carbonate is subjected to catalytic hydrogenation in the co-existence of an alkali.

4. A process for the production of an aniline derivative according to claim 3, wherein the alkali is a basic calcium salt.
